# EUROPEAN PATENT APPLICATION

(11) **EP 4 360 618 A1**
(43) Date of publication of application: **01.05.2024**
(21) Application number: 22827734.9
(22) Date of filing: 17.06.2022
(51) Int. Cl.: A61K 8/97

(54) **METHOD FOR PREPARING PURIFIED METHYL THUJATE, USE AS A FRAGRANCE COMPONENT AND COMPOSITION CONTAINING IT**

(30) Priority: 21.06.2021 ES 202130573
(71) Applicant: Eurofragance SL, 08174 Sant Cugat Del Vallès (ES)
(72) Inventor: SAN JUAN, Felipe, 08174 SANT CUGAT DEL VALLÈS (ES); VAN DEN HEUVEL, Henry, (NL)
(74) Representative: Schäfer, Matthias W.
(86) International application number: PCT/ES2022/070385
(87) International publication number: WO 2022/269114

(57) **Abstract**

Process for preparing highly concentrated, purified methyl thujate, whose chemical name is methyl 5,5-dimethylcyclohepta-1,3,5-triene-1-carboxylate, which is developed by means of low temperature crystallisation of essential oils of the Thuja species, particularly Western Red Cedar oil, use as a fragrance of the purified methyl thujate with high purity thus obtained, having a purity of more than 85%, a fragrance composition that uses said purified methyl thujate and the different uses thereof.

The present invention has the main advantage of achieving positive and long-lasting olfactory effects, as well as eliminating molecules responsible for bad odours and the ability to be used in higher concentrations in fragrances than the essential oil from which the methyl thujate is isolated.

## Description

Process for preparing highly concentrated, purified methyl thujate, whose chemical name is methyl 5,5-dimethylcyclohepta-1,3,5-triene-1-carboxylate, which is developed by means of low temperature crystallisation of essential oils of the Thuja species, particularly Western Red Cedar oil, use as a fragrance of the purified methyl thujate with high purity thus obtained, having a purity of more than 85%, a fragrance composition that uses said purified methyl thujate and the different uses thereof.

### Field of the invention

The invention relates to the field of ingredients for fragrances, especially high purity, methyl thujate, and the processes for obtaining highly concentrated methyl thujate from the essential oils of the Thuja species, in particular Western Red Cedar.

### State of the art

Today, the use of fragrance ingredients is widespread in a large number of commonly used consumer products, such as fine fragrances, personal care products, home care products, air freshener products, and the like, resulting in the perfume industry's increasing need for new fragrance ingredients that offer new olfactory benefits.

For example, fragrances are needed that are capable, in fragrance compositions, of creating interesting olfactory sensations and expanding the possibilities of creating perfumes with their new olfactory properties. Furthermore, fragrance ingredients fulfilling this primary function should preferably contribute positive properties, namely olfactory properties such as high intensity, longevity, high substantivity, odour eliminating effect or scent enhancing properties, producing fragrance enhancement and enhancement effects, in combination with other fragrance ingredients, so that novel sensory effects can be achieved from the fragrance compositions.

Methyl thujate is a product used in perfumery as a component of the essential oil extracted from Western Red Cedar trees, also known as Thuja plicata or giant arborvitae, or tree of life, as we can find described for example in JP2005162652A *"Cosmetic",* which cites Western Red Cedar extract for cosmetic use, but focusing on thujic acid purified by silica column chromatography, which is a very expensive way to purify the material, as it involves large amounts of solvent which then require evaporation, with some risk of explosion, and which is not economical for large-scale purification.

Thuja is a small genus of the Cupressaceae family comprising five species, of which Thuja plicata Donn is widely cultivated in western North America, Europe and Asia. Thuja plicata Donn is a 50-70 metre tall tree with arching branches and bright green leaves above and white stripes on the lower surface, used as a cash crop for timber in Europe and North America.

Western Red Cedar lumber is known for its resistance to insect and fungal attack. Consequently, numerous studies have been conducted on its secondary metabolites which have identified numerous interesting chemical compounds. Oil extracted from Western Red Cedar is known to repel termites and has fungicidal properties. Beta-thujaplicin is one of the ingredients of the oil, known for its antimicrobial properties, as is thujic acid, another component of the oil.

Solvent extraction of Western Red Cedar yields an essential oil with more than 100 chemical components, as we find disclosed in US200721941 *"Plants materials extraction method".* This patent discloses how methyl thujate is listed as comprising approximately 6% of the extracted oil, and with uses in perfumery, however the patent only relates to extraction processes, and does not disclose any method whereby methyl thujate can be concentrated or purified from the extracted essential oil.

Extraction methods with supercritical carbon dioxide or steam distillation are also known, as disclosed in JP2009007391 *"Antioxidant having volatility"* but due to the compounds found and their percentages it is not possible that the methyl thujate obtained has the high concentration necessary to obtain the surprising effects achieved.

Patents JP1996025846B *"Acaricide"* and JPH06329504A *"Mite and tick preventive"* show the acaricidal activity of chemical structures related to tropolones and thujate esters (Structure II), including methyl thujate. Both tropolones and thujate esters are extracted from Thuja plicata Donn by steam distillation, solvent extraction or carbon dioxide extraction. The documents admit that the compounds identified by the claimed structures, and as used, need not be pure, but only components within the extract.

Likewise, patent US4159314 *"Insect repellent animal collar"* discloses an insect repellent collar for animals consisting of a strip of polymeric material impregnated with a thujate derivative, which is described as thujic acid, thujate ester, thujate and thujaplicin. A table in the document indicates that methyl thujate comprises 21% of a given Western Red Cedar oil and 0.17% of all wood. The patent also mentions the *book"*Perfume and Fragrance Chemicals" by S. Arctander, vol. 2, references 2940 and 2941. However, these references are only for thujone and Thule alcohol, not for methyl thujate. Methyl thujate is not mentioned in Arctander's book. Although the thujate derivative is claimed to be a thujate ester and, specifically, methyl thujate, the patent provides no source of methyl thujate, no method for preparing any thujate ester, and no means of concentrating or purifying methyl thujate from Western Red Cedar oil.

Gardner and Barton (Can J Chem Vol 36 (1958) page 1612) report the separation of crude methyl thujate after steam distillation of Western Red Cedar wood chips and extraction of the oil in chloroform and extraction with 1 N sodium hydroxide solution. They give no indication of purity and the use of chloroform, a hazardous solvent requiring very careful handling and disposal, makes this process very impractical for commercial purification.

In *"*Terpenes and terpenoids IV - Some esters and amides of thujic acid' (Canadian Journal of Chemistry vol. 51, p. 3230-3235, (1973)) Hach and McDonald discuss the presence of methyl thujate in Western Red Cedar oil, but only describe the synthesis and insect repellent properties of other thujic acid esters and amides. Esters are synthesised via acid chloride, or acyl chloride.

We also find patent US20090283473 *"Plant materials extraction method"* which states that methyl thujate can be used as an ingredient in fragrances or as a fragrance in other products, but without describing the surprising effects achieved presented in this invention or achieving the high concentration necessary for it. In addition, the document states that extracts 1A28 or 1C36 can be further refined or purified but without giving details of how this could be achieved or what effects would be obtained in such a case. Initial extraction of Western Red Cedar ligneous material requires solvent extraction using explosion-proof apparatus. This is a serious problem since most industrial processes try to avoid explosion-proof installations due to the economic costs and high risks involved in such installations.

We found that the current state of the art does not show any process to obtain methyl thujate with high concentration levels that optimise it for use in fragrances from thuja plicata oil, nor does it describe its use with such high concentration or the surprising effects obtained.

### Description of the invention

In order to resolve the problem existing at present in the manufacture of fragrances, and in obtaining purified methyl thujate from thuja plicata oil with high levels of concentration, for use in fragrances, improving the current state of the art, the process for preparing purified methyl thujate has been devised, which allows achieving a high purity range, above 85%, and which is performed by crystallisation of essential oil of the Thuja species, preferably Western Red Cedar oil, and comprises, at least, a cooling stage of the essential oil at a temperature between +15°C and -80°C, preferably between +5°C and -30°C, allowing a crystallisation stage of that essential oil already cooled, or during the cooling stage, followed by a filtration and washing stage.

Optionally, it may also comprise a stage of mixing the essential oil of the Thuja species with solvent or combinations of solvents, preferably solvents totally miscible in water, performed prior to each cooling stage. In this specification, fully miscible means capable of mixing in a clear single phase liquid in all proportions of 1 part to 99 parts solvent with 99 parts to 1 part water by weight.

The crystallisation stage of the already cooled essential oil, or the first crystallisation stage in cases where there are several, may optionally comprise one or more controlled nucleations to initiate crystallisation of the already cooled essential oil, controlled nucleation being understood in this description as any process comprising seeding by introducing external material, such as particles, including crystals of methyl thujate obtained previously by this or another process, in a small proportion, as well as mechanical action, such as scratching the container.

The washing stage uses water-miscible solvents or mixtures of water-miscible solvents, optionally precooled, for example, to a temperature similar to that used for the crystallisation phase, preferably comprising water and C1 to C3 alcohols. It is envisaged that, optionally, the washing step also includes a separation of the obtained crystals of purified methyl thujate, as a solid part, from the liquid part.

It is anticipated that, optionally, each cooling stage also performs a stirring of the essential oil, or of its mixture with solvents as the case may be.

Any or all of the stages: mixing stage if applicable, cooling stage, crystallisation stage and washing stage, are repeated as many times as necessary until the desired degree of purity of the purified methyl thujate is reached.

At the end of the process, a drying stage of the purified methyl thujate crystals may optionally be included, and, also optionally, a finishing stage of the product, which may be grinding, thus obtaining a purified methyl thujate composition in powder form, or dissolution of the purified material defined above in a solvent to be used in a pre-dissolved and diluted state.

This invention also comprises the use of purified methyl thujate, obtained by that process, as a fragrance component, having the highly concentrated, purified methyl thujate (5,5-dimethylcyclohepta-1,3,5-triene-1-carboxylate methyl), with a purity range between 85 and 100%. It has been experimentally demonstrated that the use of purified methyl thujate at such a high concentration produces surprising and unexpected effects when used in the composition of fragrances.

This invention also comprises a fragrance composition using purified methyl thujate, obtained by that process, with the range of purity indicated above, and comprising
- between 0.01 % and 15.00%, with respect to the total weight, of purified methyl thujate,
- between 85.00% and 99.99%, with respect to the total weight, of aromatic components for use in perfumery and cosmetics
- optionally, one or more additional components, such as perfumery compatible solvents, UV stabilisers, odour counteractants, antioxidants, metal sequestrants, surfactants and equivalents.

This fragrance composition using purified methyl thujate is preferably used in a consumer product chosen from the group consisting of fine fragrances, household cleaning products, air care products, cosmetic products, personal care products, laundry detergents and conditioners, household cleaners, carpet cleaners, air fresheners, textiles and soft furnishings, deodorising or odour eliminating products, odour control products and scented articles.

### Advantages of the invention

The presented invention provides the main advantage that, by using purified methyl thujate with such a high concentration in the fragrance composition, surprising and unexpected effects are produced, achieving outstanding characteristics of high application in the perfumery industry.

The advantage that purified methyl thujate with such a high concentration has an intense odour that can be used in perfumery applications should be highlighted.

It is also important to note that it has also been experimentally proven to react with compounds responsible for bad odours, of the amine and thiol type, so it can be applied in consumer products, combined with its fragrance properties.

Another important advantage we should highlight is that, surprisingly, purified methyl thujate with such a high concentration makes fragrances containing it preferred, in contrast to fragrances containing the same dosage of methyl thujate dosed as part of Western Red Cedar oil, as demonstrated below in Example 3.

Another advantage is that purified methyl thujate with such a high concentration can be used in higher concentrations in fragrances than when it is commonly used as a component of Western Red Cedar oil, due to the phenolic or other odours of the oil components.

Neither should it be forgotten that purified methyl thujate with such a high concentration has been experimentally proven to reduce fragrance degradation caused by phenolic compounds within the whole Western Red Cedar oil due to interactions, for example, between iron and hinokitiol or phenolic oxidation, which favours longer fragrance duration and greater effect.

In addition, the process for preparing purified methyl thujate presented here provides multiple advantages over those currently available, the most important being the possibility of obtaining methyl thujate from thuja plicata oil by crystallisation at low temperature, obtaining purities higher than 85% of methyl thujate, much higher than those obtained at present.

### Description of the figures

In order to better understand the object of this invention, the plan attached represents a preferred practical embodiment of the method for preparing purified methyl thujate.
Figure -1- shows a simplified block diagram of said process, with the solid lines indicating the essential embodiments and the dotted lines the optional embodiments, steps or iterations.
Figure 2 shows a representation of the molecular structure of methyl thujate.
Figure 3 shows the gas chromatographic analysis obtained by the method described in Example 1 on Western Red Cedar oil.
Figure 4 shows the gas chromatography analysis obtained by the method described in Example 1 on the methyl thujate purified according to the process of the invention, showing the degree of purification of the methyl thujate obtained with respect to Western Red Cedar oil.
Figure 5 shows the results of the preference test corresponding to Example 3.

### Preferred embodiment of the invention

The constitution and characteristics of the invention may be better understood with the following description made with reference to the attached figures.

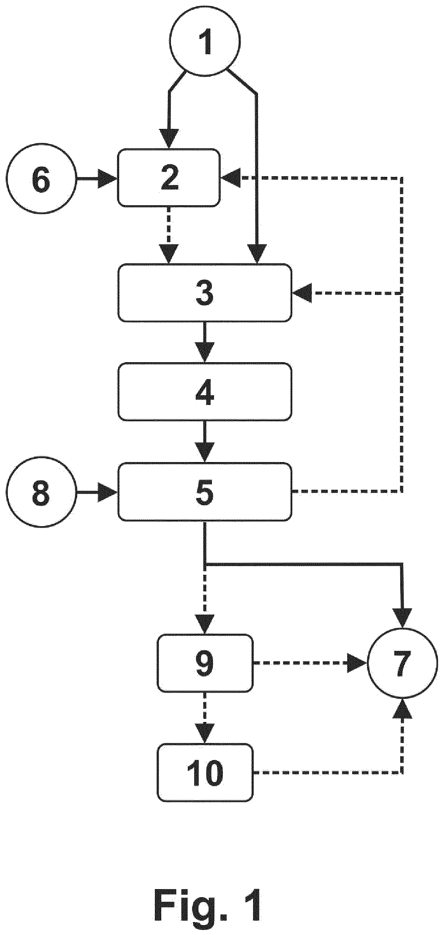

As can be seen in Figure 1, the process for preparing purified methyl thujate is illustrated, which allows achieving a high purity range, higher than 85%, and which is performed by crystallisation of essential oil (1) of the Thuja species, preferably Western Red Cedar oil, and comprises, at least, a cooling stage (3) of the essential oil (1) at a temperature between +15°C and -80°C, preferably between +5°C and -30°C, which allows a crystallisation stage (4) of said essential oil (1) already cooled, followed by a filtration and washing stage (5), although alternatively the crystallisation stage (4) could also be carried out at the same time as the cooling stage (3), before the essential oil (1) reaches the final temperature. Therefore, the cooling stage (3) and the crystallisation stage (4) can be performed consecutively, or fully or partially simultaneously.

It is envisaged that, optionally, it may also comprise a mixing stage (2) of the essential oil (1) of the Thuja species with solvent or solvent mixtures (6), performed prior to each cooling stage (3). This solvent or combination of solvents (6) are preferably fully water-miscible solvents, for example, but not limited to, deionised or distilled water, C1 to C3 alcohols, etc. preferably in a ratio comprised between 1:2 and 1:4 by weight.

The crystallisation stage (4) of the already cooled essential oil (1), or the first crystallisation stage (4) in cases where there are several, may optionally comprise one or more controlled nucleations to initiate the crystallisation of the already cooled essential oil (1), understanding, in this description, controlled nucleation to be any process comprising seeding by the introduction of external material, such as particles, including methyl thujate crystals previously obtained by this or another process, in a reduced proportion, as well as mechanical action, such as for example scratching of the container. Controlled nucleation could also optionally be performed, at least once, while cooling the mixture.

It is envisaged that, optionally, each cooling stage (3) also comprises a stirring of the essential oil (1), or of its mixture with solvents where appropriate.

The washing stage (5) comprises the use of water-miscible solvents or water-miscible solvent mixtures (8), optionally precooled, for example to a temperature similar to that used for the crystallisation phase, preferably incorporating water and C1 to C3 alcohols. It is envisaged that, optionally, the washing step (5) includes a separation of the obtained crystals of purified methyl thujate (7), as a solid part, from the liquid part.

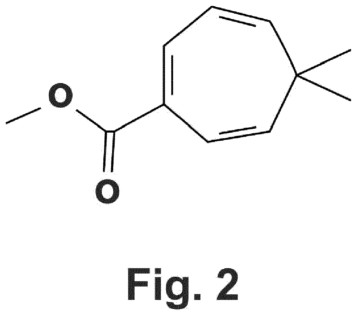

Any or all of the stages: mixing stage (2) if applicable, cooling stage (3), crystallisation stage (4) and washing stage (5), are repeated as many times as necessary until the desired degree of purity is reached in the purified methyl thujate (7), the molecular structure of which is shown in Figure 2.

At the end of the process, once the remaining steps have been carried out the required number of times, a drying step (9) of the purified methyl thujate crystals (7) may optionally be included, which may involve the use of reduced pressure, and, also optionally, a finishing stage (10) of the product. This finishing stage (10) of the product can be a grinding process, thus obtaining a purified methyl thujate composition (7) in powder form, or a dissolution process in a solvent, such as dipropylene glycol (DPG) or other usual solvents for fragrance materials, in order to be used in a pre-dissolved and diluted state.

This purified methyl thujate (7) obtained, with the necessary purity exceeding 85%, may comprise, among other compounds, methyl myrtenate (methyl-6,6-dimethylbicyclo[3.1.1]hept-3-ene-4-carboxylate) in a ratio with methyl thujate of less than 1:50.

This invention also comprises the use of purified methyl thujate, obtained by that process, as a fragrance component, the purified methyl thujate (whose chemical name is 5,5-dimethylcyclohepta-1,3,5-triene-1-carboxylate methyl, represented by the chemical structure of Figure 2) having a purity range between 85 and 100%, as defined in Example 1, preferably between 90 and 100%, and more preferably between 91 and 96%.

The purified methyl thujate may still contain some impurities. These impurities comprise between 0 and 15% of the total peak area of the chromatogram, preferably between 0 and 10% of the total peak area of the chromatogram, preferably between 4 and 9% of the total peak area of the chromatogram. These impurities are important because they can contribute to the olfactory properties of the purified methyl thujate.

This purified methyl thujate (7) obtained, with the necessary purity exceeding 85%, may comprise, among other compounds, methyl myrtenate (6,6-dimethylbicyclo[3.1.1]hept-3-ene-4-carboxylate, CAS No. 30649-97-9) in a ratio with methyl thujate of less than 1:50, preferably less than 1:250. This ratio makes it possible to distinguish between the presence of methyl thujate in a fragrance added as purified methyl thujate of this invention and the methyl thujate that may have been added as a component of Western Red Cedar oil.

This invention also comprises a fragrance composition using purified methyl thujate, obtained by that process, with the purity range indicated above, comprising
- between 0.01 % and 15.00%, with respect to the total weight, of purified methyl thujate,
- between 85.00% and 99.99%, with respect to the total weight, of aromatic components for use in perfumery and cosmetics
- optionally, one or more additional components, chosen from the group consisting of perfumery-compatible solvents, UV stabilisers, odour counteractants, antioxidants, metal sequestrants, surfactants and equivalents.

This fragrance composition using purified methyl thujate is preferably used in a consumer product chosen from the group consisting of fine fragrances, household cleaning products, air care products, cosmetic products, personal care products, laundry detergents and conditioners, household cleaners, carpet cleaners, air fresheners, textiles and soft furnishings, deodorising or odour eliminating products, odour control products and scented articles.

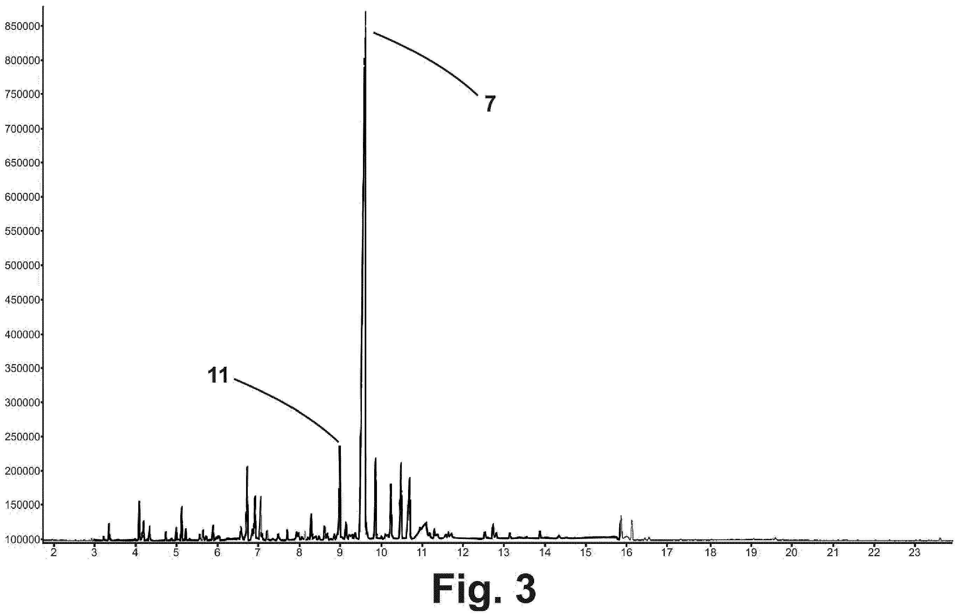

As we have seen above, there are different botanical species that give rise to cedarwood essential oils. Western red cedar oil is derived from the Thuja subspecies of the Cupressaceae family. Variations in the exact types of plants, growing conditions, location and seasonal variations can affect the composition of the extracted essential oil. Different parts of the plant: leaf, bark, heartwood and root can also give rise to oils with different compositions. Extraction methods can also affect the composition of the extracted oil. For the sake of clarity of this specification, any essential oil, regardless of how it is extracted and from which parts of the plant of Thuja species that contains more than 10% by weight of methyl thujate, is considered to be Western Red Cedar oil for the purposes of this specification. The percentage of methyl thujate present in an oil can be estimated by measuring the areas under the various peaks obtained from a gas chromatography (GC) analysis of an oil using the method described in Example 1 and represented in Figure 3. The sum of the area values of all peaks and their normalisation to 100% of the total area of the chromatogram allows the calculation of the individual percentages of the peak area. By adding these values for all peaks other than methyl thujate and subtracting them from 100%, a value is calculated for the percentage of methyl thujate.

It is understood that the word "fragrance" is synonymous with the word "perfume", as are the terms "fragrance composition" or "perfume composition", and that it relates to a mixture of materials with olfactory activity that provides a pleasant odour. The term "fragrance ingredient", which is also synonymous with the terms "fragrance component", "perfume ingredient" and "perfume component", is understood to mean any individual material that may be an ingredient within a fragrance composition, even though that perfume ingredient may itself comprise many individual organic chemical compounds and have a pleasant odour. The term chemical compound is defined as any substance composed of identical molecules consisting of atoms of two or more chemical elements. Chemical compounds can exist as structural, geometric and optical isomers. Reference to any chemical compound by name will be interpreted in the broadest context; for example, thujaplicin will include all isomeric forms of the molecule unless specifically stated otherwise, while β-thujaplicin refers to that specific isomer and not to the other isomers of thujaplicin.

A wide variety of odoriferous materials are known for use in perfumery, including materials such as alkenes, alcohols, aldehydes, ketones, esters, ethers, nitriles, amines, oximes, acetals, ketals, thiols, thioketones, imines, etc.

The fragrance compositions of the present invention may optionally contain other natural extracts, such as essential oils. Natural extracts are produced by subjecting the appropriate natural materials, such as plant components: leaves, flowers, seeds, roots or stems, to an extraction process. The extraction processes are well known to persons skilled in the art and many of them are described in The Essential Oils by E Guenther, published in 1949 by D van Nostrand. The essential oils can undergo additional processes to rectify and purify the oils, for example, by removing the terpene components by "head cutting" and/or removing the wax components by "tail cutting". These natural extracts include, among others, those obtained from citrus species such as: lemons, oranges, tangerines, grapefruit, ugli fruit; from spices such as anise, cinnamon cloves, or herbs such as basil, mint, lavender, lavandin, thyme, rosemary, or from many varieties of plants such as geranium, various roses, citronella, cypress, eucalyptus, balsam of Peru, camphor, sandalwood, ylang and cedar and mixtures thereof.

Perfume compositions may be relatively simple in composition with a minimum of two perfume or fragrance ingredients, including purified methyl thujate, or may comprise very complex mixtures of natural and synthetic chemical compounds chosen to provide any desired odour. For creativity, it is preferred that the perfume compositions contain more than 5 perfume ingredients, and it is more preferable that the perfume compositions contain more than 10 perfume ingredients. Perfume ingredients are described in more detail in S. Arctander, Perfume Flavors and Chemicals. Vol. I and II, Montclair, N.J. and in Allured's Flavor and Fragrance Materials-2007 ISBN 978-1-93263326-9 by Allured Publishing Corporation. Without limitation, examples of perfume ingredients may include:
hydrocarbons such as 3-carene; α-pinene; beta-pinene; alpha-terpinene; gamma-terpinene; p-cymol; bisabolene; camphene; caryophyllene; cedrene; farnesene; limonene; longifolene; myrcene; ocimene; valencene; (E,Z)-1,3,5-undecatriene;
aliphatic alcohols, such as hexanol; octanol; 3-octanol; 2,6-dimethylheptanol; 2-methylheptanol, 2-methyloctanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methylheptan-2-ol; (E,Z)-2,6-nonadienol; 3,7-dimethyl-7-methoxyoctan-2-ol; 9-decenol; 10-undecenol; 4-methyl-3-decen-5-ol;
aliphatic aldehydes and their acetals such as hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanal diethyl acetal; 1,1-dimethoxy-2,2,5-trimethyl-4-hexene; citronellyl oxyacetaldehyde
aliphatic ketones and their oximes, such as 2-heptanone; 2-octanone; 3-octanone; 2-nonanone; 5-methyl-3-heptanone; 5-methyl-3-heptanonoxime; 2,4,4,7-tetramethyl-6-octen-3-one;
aliphatic sulphur compounds such as 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
Aliphatic nitriles such as 2-nonenonitrile; 2-tridecenonitrile; 2,12-tridecenonitrile; 3,7-dimethyl-2,6-octadienonitrile; 3,7-dimethyl-6-octenonitrile;
aliphatic carboxylic acids and their esters, such as (E)- and (Z)-3-hexenyl formiate; ethyl acetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octan-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl-2-methyl pentanoate; ethyl hexanoate; allyl hexanoate; ethyl heptanoate; allyl heptanoate; allyl heptanoate; allyl heptanoate; ethyl octanoate; ethyl (E,Z)-2,4-decadienoate; methyl 2-octinate; methyl 2-noninoate; allyl 2-isoamyl oxyacetate; methyl 3,7-dimethyl-2,6-octadienoate;
terpene alcohols such as citronellol, geraniol, nerol, linalool, lavadulol, nerolidol, farnesol, tetrahydrolinalool and tetrahydrogeraniol; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglates and 3-methyl-2-butenoates thereof;
terpene aldehydes and ketones such as geranial; neral; citronellal; 7-hydroxy-3,7-dimethyloctanal; 7-methoxy-3,7-dimethyloctanal; 2,6,10-trimethyl-9-undecenal; geranyl acetone; and the dimethyl and diethyl acetals of geranial, neral and 7-hydroxy-3,7-dimethyloctanal;
cyclic terpene alcohols such as menthol; isopulegol; a-terpineol; terpinenol-4; menthan-8-ol; menthan-1-ol; menthan-7-ol; borneol; isoborneol; linalool oxide; nopol; cedrol; ambrinol; vetiverol; guaiol; and their formates, acetates, propionates, isobutyrates, butyrates, isovalerates, pentanoates, hexanoates, crotonates, tiglates and 3-methyl-2-butenoates;
cyclic terpene aldehydes and ketones such as menthone; isomethone; 8-mercaptomentan-3-one; carvone; camphor; fenchone; a-ionone; beta-ionone; alpha-n-methylionone; beta-n-methylionone; alpha-isomethylionone; beta-isomethylionone; alpha-irone; alpha-damascone; alpha-damascenone; beta-damascone; beta-damascone; gamma-damascone; delta-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; nootkatone; dihydronootkatone; a-sinensal; beta-sinensal; methylcedrylketone;
cyclic alcohols such as 4-tert-butyl-cyclohexanol; 3,3,5-trimethylcyclohexanol; 3-isocamphylcyclohexanol; 2,6,9-trimethyl-(Z2,Z5,E9)-cyclododecatrien-1-ol; 2-isobutyl-4-methyltetrahydro-2H-pyran-4-ol; from the group of cycloaliphatic alcohols, e.g. a,3,3-trimethylcyclohexylmethanol; 2-methyl-4-(2,2,3-trimethyl-3-cyclopent-1-yl)-2-buten-1-ol; 2-methyl-4-(2,2,3-trimethyl-3-cyclopol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
cyclic ethers such as cineol; cedryl methyl ether; cyclodecile methyl ether; (ethoxymethoxy)cyclododecane; a-cedrene epoxide; 3a,6,6,9a-tetramethyldodecahydronaphthal[2,1-b]furan; 3a-ethyl-6,6,9a-tri-ethyldodecahydronaphthal[2,1-b]furan; 1,5,9-trimethyl-13-oxabicyclo[10.1,0]trideca-4,8-diene; pink oxide; 2-(2,4-dimethyl-3-cyclohexen-1-yl)-5-methyl-5-(1-methylpropyl)-1,3-dioxane;
cyclic ketones such as 4-tert-butylcyclohexanone; 2,2,5-tri n-ethyl-5-pentyl-pentanone; 2-heptylcyclopentanone; 2-pentylpentanone; 2-hydroxy-3-methyl-2-cyclopentadiene; 3-methyl-cis-2-pentadiene-1-yl-2-cyclopentadiene; 3-methyl-2-pentyl-2-cyclopentadiene; 3-methyl-2-pentyl-2-cyclopentadiene; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 9-cycloheptadecen-1-one; cyclopentadecanone; cyclohexadecanone;
cycloaliphatic aldehydes such as 2,4-dimethyl-3-cyclohexenecarbaldehyde; 2-methyl-4-(2,2,6-tri methylcyclohexen-1-yl)-2-butenal; 4-(4-hydroxy-4-methylpentyl)-3-cyclohexenecarbaldehyde; 4-(4-methyl-3-pentene-1-yl)-3-cyclohexenecarbaldehyde;
cycloaliphatic ketones such as 1-(3,3-dimethylcyclohexyl)-4-pentene-1-one; 1-(5,5-dimethyl-1-cyclohexen-1-yl)-4-pentene-1-one; 2,3,8,8-tetra methyl-1,2,3,4,5,6,7,8-octa-hydro-2-naphthalenylmethylketone; methyl-2,6,10-trimethyl-2,5,9-cyclodecatrienylketone; tert-butyl-2,4-dimethyl-3-cyclohexen-1-ylketone;
esters of cyclic alcohols such as 2-tert-butylcyclohexyl acetate; 4-tert-butylcyclohexyl acetate; 2-tert-pentylcyclohexyl acetate; 4-tert-pentylcyclohexyl acetate; decahydro-2-naphthyl acetate; 3-pentyl-tetrahydro-2H-pyran-4-yl acetate; decahydro-2,5,5,8a-tetramethyl-2-naphthyl acetate; 4,7-methane-3a,4,5,6,7,7a-hexahydro-5- or-6-indenyl acetate; 4,7-methane-3a,4,5,6,7,7a-hexahydro-5- or -6-indenyl propionate; 4,7-methane-3a,4,5,6,7,7a-hexahydro-5- or -6-indenyl isobutyrate; 4,7-methanooctahydro-5- or -6-indenyl acetate; esters of cycloaliphatic carboxylic acids such as allyl 3-cyclohexyl propionate; allyl cyclohexyloxyacetate; methyl dihydrojasmonate; methyl jasmonate; ethyl 2,3,6,6-tetramethyl-2-cyclohexenecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-ethyl acetate;
aromatic hydrocarbons such as styrene and diphenylmethane;
araliphatic alcohols such as benzyl alcohol; 1-phenethyl alcohol; 2-phenethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
esters of araliphatic alcohols and aliphatic carboxylic acids such as benzyl acetate; benzyl propionate; benzyl isobutyrate; benzyl isovalerate; 2-phenethyl acetate; 2-phenethyl propionate; 2-phenethyl isobutyrate; 2-phenethyl isovalerate; 1-phenethyl acetate; a-trichloromethylbenzyl acetate; a,a-dimethylphenethyl acetate; a,a-dimethylphenethyl butyrate; cinnamyl acetate; 2-phenoxyethyl isobutyrate; 4-methoxybenzyl acetate;
araliphatic ethers such as 2-phenethyl methyl ether; 2-phenethyl isoamyl ether; 2-phenthyl 1-ethoxyethyl ether; phenylacetaldehyde dimethyl acetal; phenylacetaldehyde diethyl acetal; hydratropic aldehyde dimethyl acetal; phenylacetaldehyde glycerol acetal; 2,4,6-trimethyl-4-phenyl-1,3-dioxane; 4,4a,5,9b-tetrahydro-roindeno[1,2-c1]-m-dioxin; 4,4a,5,9b-tetrahydro-2,4-dimethylindeno[1,2-d]-m-dioxin;
aromatic and araliphatic aldehydes such as benzaldehyde, phenylacetaldehyde, 3-phenylpropanal, hydropic aldehyde, 4-methylbenzaldehyde and 4-methylphenylacetaldehyde; 3-(4-ethylphenyl)-2,2-dimethylpropanal; 2-methyl-3-(4-isopropylphenyl)-propanal; 2-methyl-3-(4-tert-butylphenyl)propanal; 3-(4-tert-butylphenyl)propanal; cinnamaldehyde; a-butylcinnamaldehyde; a-amylcinnamaldehyde; a-hexylcinnamaldehyde; 3-methyl-5-phenylpentane; 4-methoxybenzaldehyde; 4-hydroxy-3-methoxybenzaldehyde; 4-hydroxy-3-methoxybenzaldehyde; 3-methylenedioxybenzaldehyde; 3,4-dimethoxybenzaldehyde; 2-methyl-3-(4-methoxyphenyl)propanal; 2-methyl-3-(4-methylenedioxyphenyl)propanal;
aromatic and araliphatic ketones such as acetophenone; 4-methylacetophenone; 4-methoxyacetophenone; 4-tert-butyl-2,6-dimethylacetophenone; 4-phenyl-2-butanone; 4-(4-hydroxyphenyl)-2-butanone; 1-(2-naphthalenyl)-ethanone; benzophenone; 1,1,2,3,3,6-hexamethyl-5-indanylmethylketone; 6-tert-butyl-1,1-dimethyl-4-indanylmethylketone; 142,3-dihydro-1,1,2,6-tetramethyl-3-(1-,ethylethyl)-1H-5-indenylethanone; 5',6',7',8'-tetrahydro-3',5',5',6',8',8'-hexamethyl-2-acetonephthone;
Aromatic and araliphatic carboxylic acids and their esters, such as benzoic acid; phenylacetic acid; methyl benzoate; ethyl benzoate; hexyl benzoate; benzyl benzoate; methylphenyl acetate; ethylphenyl acetate; geranyl acetate; phenylphenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycate; ethyl 3-methyl-3-phenylglycate;
nitrogen-containing aromatic compounds, such as 2,4,6-trinitro-1,3-dimethyl-5-tert-butylbenzene; 3,5-dinitro-2,6-dimethyl-4-tert-butyl-acetophenone; cinnamylnitrile; 5-phenyl-3-methyl-2-pentenonitrile; 5-phenyl-3-methylpentanonitrile; methyl anthranilate; methyl N-methylanthranilate; methyl anthranilate Schiffs bases with 7-hydroxy-3,7-dimethyloctanal, 2-methyl-3-(4-tert-butylphenyl)propanal or 2,4-dimethyl-3-cyclohexene-carbaldehyde; 6-isopropylquinoline; 6-isobutylquinoline; 6-sec-butylquinoline; indole; skatole; 2-methoxy-3-isopropylpyrazine; 2-isobutyl-3-methoxypyrazine; 4-(4,8-dimethyl-3,7-nonadienyl)pyridine;
phenols, phenyl ethers and phenyl esters such as estragole, anethole, eugenol, eugenyl methyl ether, isoeugenol, isoeugenyl methyl ether, thymol, carvacrol, diphenyl ether, beta-naphthyl methyl ether, beta-naphthyl ethyl ether, beta-naphthyl isobutyl ether, 1,4-dimethoxybenzene and eugenyl acetate; 2-methoxy-4-methylphenol; 2-ethoxy-5-(1-propenyl)phenol; p-cresylphenyl acetate; from the group of heterocyclic compounds, for example, 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
lactones such as 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene-1,12-dodecandioate; ethylene-1,13-tridecandioate; coumarin; 2,3-dihydrocontrarnarin; octahydrocoumarin.

The fragrance composition of the invention may include one or more materials such as solvents or UV stabilisers. Examples of suitable solvents include hydrocarbons such as those sold under the trade name Isopar (registered trademark); ethers such as those sold under the trade name Dowanol (registered trademark): benzyl benzoate; isopropyl myristate; dialkyl adipates; dialkyl succinates; dialkyl glutarates such as dimethyl esters sold under the trade name Flexisolv (trade name); citrate esters, such as triethyl citrate and tributyl acetyl citrate; diethyl phthalate; diethylene glycol monoethyl ether, 3-methoxy-3-methyl-1-butanol; dipropylene glycol; and isopropylidene glycerol sold under the trade name Augeo (registered trademark) Clean Multi.

Examples of UV stabilisers include butyl methoxy dibenzoyl methane; bis-ethylhexyloxyphenolmethoxyphenyltriazine; those sold under the trade name Uvinol (registered trademark) as Uvinul D50 [bis(2,4-dihydroxyphenyl)-methanone], Uvinul MC80 (ethylhexyl methoxycinnamate) and Uvinul M40 (benzophenone-3); those sold under the trade name Parsol (registered trademark), as Parsol (registered trademark). The fragrance composition of the invention may include one or more carrier materials, such as solvents or UV stabilisers.

The use of this fragrance is a consumer product chosen from the group consisting of fine fragrances, household cleaning products, air care products, cosmetic products, personal care products, laundry detergents and conditioners, household cleaners, carpet cleaners, air fresheners, textiles and soft furnishings, deodorising and odour control products and scented articles.

In this document, "consumer product" means a personal care, cosmetic, textile, household, automotive and family product intended to be used or consumed in the form in which it is sold, and not for further commercial manufacturing modification or resale. Such products include, but are not limited to, products or methods for the treatment of hair (human, dog or cat), such as bleaching, colouring, dyeing, conditioning, shampooing and styling; products applied to the skin, such as Eau de Parfum, colognes, body sprays, deodorants and antiperspirants; personal cleansing products such as bar soaps, liquid soap, shower gels, body washes and scrubs; cosmetic skin care products, including the application of creams, lotions, colour cosmetics and other topical application products; and shaving products. Textile products include those for laundry detergent and garment care products, such as powder, liquid and unit-dose detergents, laundry additives, fabric softeners, dryer sheets, ironing sprays and fabric fresheners; products for the treatment of soft household furnishings, such as carpet cleaners and odour eliminators. Hard surface products include hand and machine dishwashing products, hard surface cleaners for floors, walls, windows and mirrors, and cleaners for sinks. Other products used in home care are: air care products, such as plug-in air fresheners, aerosol products and scented candles; impregnated substrates, such as wet wipes for skin cleansing, refreshing and household cleaning, nappies, sanitary products, paper products, such as tissues and drawer liners.

Some examples and the results obtained during the experimental process are attached below.

### Example 1: Purification of methyl thujate from Western Red Cedar oil.

A solvent mixture was prepared by mixing 4.5 kg of methanol with 1.5 kg of distilled water. A sample of approximately 1.7 kg of steam distilled Western Red Cedar oil was mixed with 1.7L of the solvent mixture while stirred in a reactor capable of cooling to at least -20°C. The temperature of the mixture was reduced to -20°C, using a cooling ramp of 0.5°C/minute, with continuous stirring.

Once the temperature reached 5°C, a few crystals of methyl thujate (0.2 g in this case) were added to the mixture. The methyl thujate is then crystallised and the mixture was stirred for a further two hours after reaching -20°C, while maintaining the temperature.

The crystal mixture was filtered through a vacuum Buchner funnel with a filter paper filter. The obtained filtered solids were redispersed in 1.7 L of the methanol/water mixture at -20°C, with stirring for 30 min, and the filtration step was repeated.

The purified methyl thujate crystals were dried under vacuum for 24 hours at room temperature.

The yield of crystalline methyl thujate was approximately 40% of the original weight of Western Red Cedar oil and the purity determined by gas chromatography, as described below, was 92.7%.

The purity of methyl thujate was estimated by gas chromatography (GC) analysis with a flame ionisation detector by adding the area percentages of other peaks and deducting them from 100%. The gas chromatograph used was an Agilent model 7890 equipped with a flame ionisation detector (FID) and a 100% dimethylpolysiloxane GC column with the following dimensions: 20 metres long x 0.1 millimetres internal diameter and 0.1 micrometres film thickness. Helium was used as a carrier gas at a flow rate of 0.36 ml/min (25.8 cm/s). The injector temperature was set at 250°C. The oven temperature program used was: 80°C (at time zero), followed by a temperature program increasing at 6°C/min up to 250°C. The temperature was maintained at 250°C for 1 minute. The temperature of the flame ionisation detector was set at 300°C.

Peak Area Integration was performed using Agilent Chemstation software (version F.01.03.2357) using the following settings:

| | |
|---|---|
| - Area reject | 3000 |
| - Peak width | 0.06 |
| - Shoulder detection | off |
| - Threshold | 12 |
| - Integration OFF | 0.01 |
| - Integration ON | 4 |

A sample of approximately 1 g of dried crystals of purified methyl thujate was dissolved in 9 g of ethanol. 0.1 ul of this dilution of methyl thujate in ethanol was injected into the GC using the split mode (1:100) by means of an autosampler. A chromatogram similar to Figure 2 was produced and the areas of all peaks are added together and normalised to 100%. The values of all minor peaks are then added together and subtracted from 100% to obtain the percentage purity of methyl thujate.

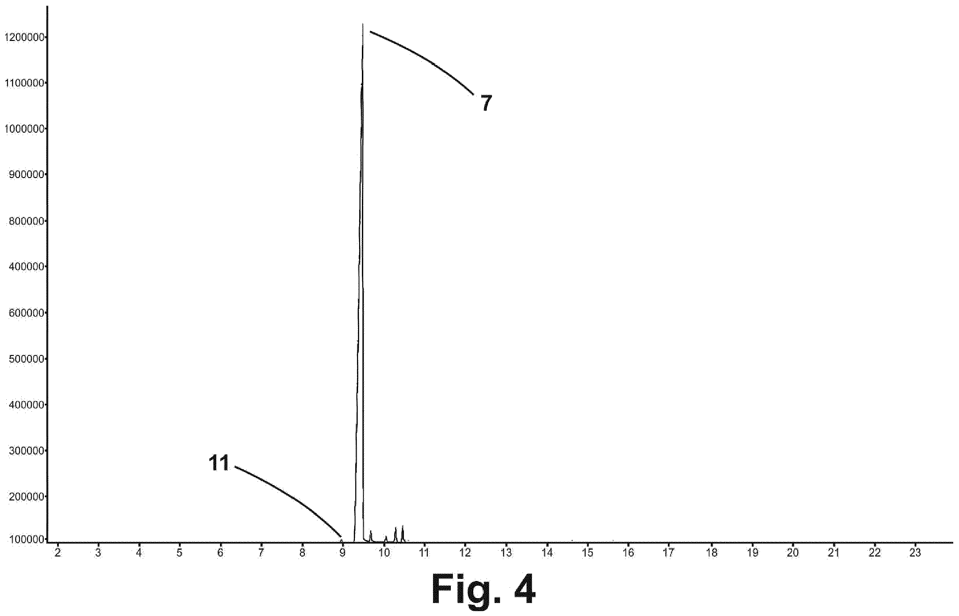

Figure 3 shows the gas chromatographic analysis thus obtained on the starting Western Red Cedar oil, and Figure 4 shows the gas chromatographic analysis obtained on the purified methyl thujate. Comparison of these chromatograms clearly demonstrates the degree of purification of methyl thujate obtained by the crystallisation process described.

A peak for methyl thujate (7) and methyl myrtenate (11) is identified in both chromatograms, showing the percentage area ratio of these peaks.

As explained above, to distinguish between the presence of methyl thujate in a fragrance added as purified methyl thujate of this invention and methyl thujate that may have been added as a component of Western Red Cedar oil, we can study the ratio of methyl thujate and methyl myrtenate (MM). The MM elutes at 8.9 minutes under the gas chromatographic conditions of Example 1 on a 20 m DB1 column. In typical commercial Western Red Cedar oil, the ratio of MM to methyl thujate is greater than 1:20, but in the purified methyl thujate of the invention the ratio is about 1:1000.

### Example 2: Perfume compositions containing methyl thujate.

**Table 1 - 2A Fragrance for Men (Eau de Toilette) (12%)**

| **Ingredient** | **Quantity (g)** |
|---|---|
| Ambrocenide | 1.5 |
| Ambroxan | 15 |
| Benzoin Abs. 50 | 10 |
| Benzyl salicylate | 50 |
| Italian bergamot oil | 50 |
| Calone | 1.5 |
| Canthoxal | 2.5 |
| Ceylon cardamom oil | 1.5 |
| Texas cedar oil | 30 |
| Cis-3-hexenyl salicylate | 100 |
| Laevo-Citronellol | 10 |
| Evernyl | 5 |
| Hedione | 100 |
| Iso E Super | 350 |
| Laevo-Muscone | 5 |
| Linalool Synth. | 15 |
| Linalyl acetate | 15 |
| Magnolan | 8.5 |
| Methoxymelonal | 2.5 |
| Methyl Pamplemousse | 30 |
| Lily-of-the-valley base | 30 |
| Indonesian nutmeg oil | 7 |
| Indonesian Patchouli Oil Rect. | 20 |
| Black pepper oil | 15 |
| Prenyl salicylate | 5 |
| Vetiver Haiti oil | 20 |
| Dipropylene glycol | 93 |
| ***METHYL THUJATE*** | ***7*** |
| Total parts | 1000 |

**Table 2 - Fragrance 2B for shower gel (1%)**

| **Ingredient** | **Quantity (g)** |
|---|---|
| Aldehyde C-12 Lauric | 1 |
| Aldehyde Supra | 1 |
| Ceylon cardamom oil | 1 |
| Cetalox | 2.5 |
| Cuminaldehyde (10%) | 3 |
| Damascone, alpha | 2.5 |
| Eucalyptol | 40 |
| Eucalyptus Globulus oil | 60 |
| Pure galaxolide | 20 |
| Geraniol | 60 |
| Egyptian geranium oil | 70 |
| Geranyl acetate | 5 |
| Hydroxyambran | 1.5 |
| Super Lavandin Oil | 80 |
| Linalool Synth. | 5 |
| Menton | 5 |
| American peppermint oil | 60 |
| Rosyrane Super | 5 |
| Terpineol Extra | 10 |
| Dipropylene glycol | 566.5 |
| ***METHYL THUJATE*** | ***1*** |
| Total parts | 1000 |

### Example 3: Sample evidence of the olfactory benefit of purified methyl thujate compared to the equivalent contained in Western Red Cedar oil or with the absence of methyl thujate.

Composition 2A in Table 1 of Example 2 was considered to study the sensory effect of the material methyl thujate and the effect of adding it in pure form or as a component of Thuja Plicata oil. A classification test was performed to evaluate this sensory assessment.

In this study, three alternative compositions were constructed from the initial formula:
- Alternative formula with X grams of methyl thujate (version 1)
- Alternative formula without methyl thujate. X grams of dipropylene glycol were included as a substitute (version 2)
- Alternative formula with an equivalent amount of thuja plicata oil. It means the amount of oil containing X grams of methyl thujate. Considering an average methyl thujate concentration of 55% in Thuja Plicata oil, the equivalent weight of oil added in the formula is X / 0.55 g. The necessary grams of dipropylene glycol are eliminated from the initial formula to maintain 1000 g total weight (version 3)

**Table 3 - Three alternative compositions derived from composition 2A of Example 2.**

| | Quantity (g) | | |
|---|---|---|---|
| **Ingredient** | **Composition A1** | **Composition A2** | **Composition A3** |
| Ambrocenide | 1.5 | 1.5 | 1.5 |
| Ambroxan | 15 | 15 | 15 |
| Benzoin Abs. 50 | 10 | 10 | 10 |
| Benzyl salicylate | 50 | 50 | 50 |
| Italian bergamot oil | 50 | 50 | 50 |
| Calone | 1.5 | 1.5 | 1.5 |
| Canthoxal | 2.5 | 2.5 | 2.5 |
| Ceylon cardamom oil | 1.5 | 1.5 | 1.5 |
| Texas cedar oil | 30 | 30 | 30 |
| Cis-3-hexenyl salicylate | 100 | 100 | 100 |
| Laevo-Citronellol | 10 | 10 | 10 |
| Evernyl | 5 | 5 | 5 |
| Hedione | 100 | 100 | 100 |
| Iso E Super | 350 | 350 | 350 |
| Laevo-Muscone | 5 | 5 | 5 |
| Linalool Synth. | 15 | 15 | 15 |
| Linalyl acetate | 15 | 15 | 15 |
| Magnolan | 8.5 | 8.5 | 8.5 |
| Methoxymelonal | 2.5 | 2.5 | 2.5 |
| Methyl Pamplemousse | 30 | 30 | 30 |
| Lily-of-the-valley base | 30 | 30 | 30 |
| Indonesian nutmeg oil | 7 | 7 | 7 |
| Indonesian Patchouli Oil Rect. | 20 | 20 | 20 |
| Black pepper oil | 15 | 15 | 15 |
| Prenyl salicylate | 5 | 5 | 5 |
| Vetiver Haiti oil | 20 | 20 | 20 |
| Dipropylene glycol | *93* | *100* | *87.3* |
| **METHYL THUJATE** | *7* | *0* | *0* |
| Thuja Plicata Oil | *0* | *0* | *12.7* |
| ***Total grams*** | 1000 | 1000 | 1000 |

Each composition was applied in Eau de Toilette at 12%. These samples were applied to strips of paper for smelling. They were evaluated at two different times: initial time (t0) and after 6 hours after application (6h). This was done to evaluate the effect of methyl thujate over time.

A panel of 10 experts was considered for the sensory evaluation. The panellists had to smell the three samples and rank them according to their personal preference, taking into account the type of product they were smelling.

For each panellist, the first-ranked sample scored 3, the second-ranked sample scored 2, and the last-ranked sample scored 1. When all panellists had ranked the samples, the scores assigned to each sample were added together. The samples were then tested for significant differences using Friedman's test and calculating the least significant difference. Score sums were compared between all possible pairs of samples. A significance level of 95% was considered.

**Table 4 - Men's Fragrance Results (Eau de Toilette)**

| | **t0** | **2h** | **6h** |
|---|---|---|---|
| Composition A1 | 25.0 | 26.0 | 29.0 |
| Composition A2 | 22.0 | 14.0 | 15.0 |
| Composition A3 | 13.0 | 20.0 | 16.0 |

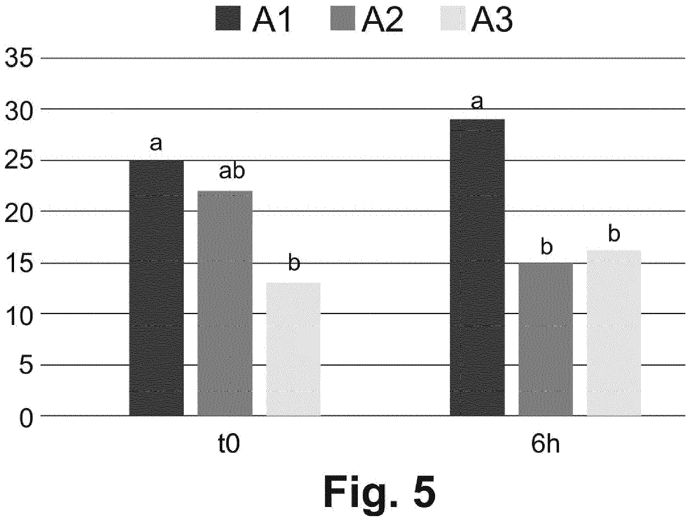

In the preference ranking test (sum of scores) in Figure 5, where results with a different letter mean significant differences (95%) and results with the same letter mean no significant differences (95%), as can be seen, samples containing purified methyl thujate scored significantly higher compared to the sample without methyl thujate after 6 hours of perfume application. In addition, purified methyl thujate allows a significantly more appreciated odour at T0 and after 6 hours compared to methyl thujate added as part of Thuja Plicata oil. These observations point to the benefit of using purified methyl thujate as a fragrance ingredient. This benefit becomes more evident over time.

A similar study was conducted with fragrance B from example 2, applied in the shower gel. The results showed a similar trend and conclusions.

### Example 4 Odour control with methyl thujate (amine).

A sample of approximately 1 g of dried crystals of purified methyl thujate was dissolved in 8 g of diethyl phthalate in a 10 ml volumetric flask. 0.1 g of hexylamine was added to the solution and more diethyl phthalate was added to bring the solution to the 10 mL mark. A reference sample was prepared by dissolving 0.1 g of hexylamine in 10 mL of diethyl phthalate in a volumetric flask. Both samples were stirred to form a homogeneous solution. The solution mixtures were allowed to react for 30 minutes at room temperature.

Sample and reference aliquots were then placed in separate 1mL sample vials and analysed by GC using an autosampler for injection and with FID detection (GC is Agilent 7890 series), with the following conditions:
Injection: 1 ul in split mode (1:1000) through the GC autosampler.
Injector temperature 250°C
Column: DB-1 (100% dimethylpolysiloxane) 20 m x 0.1 mm x 0.1 um
Carrier gas: Helium at 0.36 ml/min (linear velocity 25.8 cm/s)
Oven program: initial temperature 80°C; rising linearly 6°C/min up to an upper limit of 110°C, then increasing at 20°C/min up to 250°C which is maintained for 20 minutes.
FID detector temperature 300°C

The integration was carried out using Agilent Chemstation software version F.01.03.2357 with the following values:

| | |
|---|---|
| - Area reject | 3000 |
| - Peak width | 0.06 |
| - Shoulder detection | off |
| - Threshold | 12 |
| - Integration OFF | 3 minutes |

Two replicate experiments were performed simultaneously and GC analyses of each sample were triplicated. The hexylamine peak area was measured for the test samples and external references and the hexylamine reduction due to reaction with methyl thujate was calculated as a percentage of the averaged peak areas. There was a 68% reduction of hexylamine, which experimentally demonstrates the ability of methyl thujate to react with amines giving rise to a non-odorous product and thus reduce bad odours.

### Example 5 Odour control with methyl thujate (thiol).

A sample of approximately 1 g of dried crystals of purified methyl thujate was dissolved in 8 g of diethyl phthalate in a 10 ml volumetric flask. 0.1 g of butylthiol was added to the solution and more diethyl phthalate was added to complete the solution to the 10 mL mark. A reference sample was prepared by adding 0.1 g of butyl thiol to 10 mL of diethyl phthalate in a 10 mL volumetric flask. Both samples were stirred to form a homogeneous solution. The solution mixtures were allowed to react for 2 hours at room temperature.

Aliquots of the samples and reference were then placed in separate 1 mL sample vials and analysed by GC using an autosampler for injection with FID detection under the same conditions described in Example 4.

As in Example 4, two replicate experiments were performed simultaneously and GC analyses of each sample were triplicated. The butylthiol peak area was measured for the test samples and external references and the butylthiol reduction due to reaction with methyl thujate was calculated as a percentage of the averaged peak areas. There was a 13% reduction in butylthiol, which experimentally demonstrates the ability of methyl thujate to react with thiols and thus reduce bad odours.

Persons skilled in the art will easily understand that the characteristics of different embodiments can be combined with characteristics of other possible embodiments whenever such a combination is technically possible.

All the information referring to examples or modes of embodiment, form part of the description of the invention.

## Claims

1. - Process for preparing purified methyl thujate **characterised in that it** is performed by crystallisation of essential oil (1) of the Thuja species, and comprises, at least, a cooling stage (3) of the essential oil (1) at a temperature between +15°C and -80°C, a crystallisation stage (4) of the essential oil (1), and a washing stage (5) subsequent to the crystallisation stage (4).

2. - Process for preparing purified methyl thujate, according to the preceding claim, **characterised in that it** comprises a mixing stage (2) of the essential oil (1) of the Thuja species with solvents or combinations of solvents (6), performed prior to each cooling stage (3).

3. - Process for preparing purified methyl thujate, according to any of the preceding claims, **characterised in that** the crystallisation stage (4) of the essential oil (1), or the first crystallisation stage (4) in cases where there are several, comprises at least one controlled nucleation to initiate the crystallisation of the essential oil (1).

4. - Process for preparing purified methyl thujate, according to any of the preceding claims, **characterised in that** the washing step (5) comprises the use of water-miscible solvents or water-miscible solvent mixtures (8) incorporating water and C1 to C3 alcohols.

5. - Process for preparing purified methyl thujate, according to any of the preceding claims, **characterised in that** the washing step (5) comprises a separation of the obtained crystals of purified methyl thujate (7), as a solid part, from the liquid part.

6. - Process for preparing purified methyl thujate, according to any one of the preceding claims, **characterised in that** each cooling stage (3) also comprises a stirring of the essential oil (1) or its mixture.

7. - Process for preparing purified methyl thujate, according to any of the preceding claims, **characterised in that** any or all of the stages: mixing stage (2), cooling stage (3), crystallisation stage (4) and washing stage (5) are repeated as many times as necessary until the desired degree of purity of the purified methyl thujate is reached.

8. - Process for preparing purified methyl thujate, according to any of the preceding claims, **characterised in that it** comprises a drying step (9) of the purified methyl thujate crystals (7), which is executed once the remaining steps have been carried out the required number of times.

9. - Process for preparing purified methyl thujate, according to any of the preceding claims, **characterised in that it** comprises a finishing stage (10) of the product, which is executed once the remaining stages have been performed the required number of times, and which is chosen from the group formed by grinding process and dissolution process in a solvent usual for fragrance materials.

10. - Use of purified methyl thujate obtained according to the process described in any one of the preceding claims as a fragrance component, **characterised in that** the purified methyl thujate has a purity range from 85 to 100%.

11. - Use of purified methyl thujate as a fragrance component, according to claim 10, **characterised in that** the purified methyl thujate has a purity range of 90 to 100%.

12. - Use of purified methyl thujate as a fragrance component, according to claim 11, **characterised in that** the purified methyl thujate has a purity range of 92 to 97%.

13. - Fragrance composition using purified methyl thujate, according to any one of the preceding claims, **characterised in that it** comprises
- between 0.01% and 15.00%, with respect to the total weight, of purified methyl thujate, and
- between 85.00% and 99.99%, with respect to the total weight, of aromatic components for use in perfumery.

14. - Fragrance composition using purified methyl thujate, according to claim 13, **characterised in that it** comprises one or more additional components, chosen from the group consisting of perfumery compatible solvents, UV stabilisers, odour counteractants, antioxidants, metal sequestrants, surfactants and equivalents.

15. - Use of a fragrance composition using purified methyl thujate according to any one of claims 13 to 14, **characterised in that** the fragrance composition is used in a consumer product chosen from the group consisting of fine fragrances, household cleaning products, air care products, cosmetic products, personal care products, laundry detergents and conditioners, household cleaners, carpet cleaners, air fresheners, textiles and soft furnishings, deodorising or odour eliminating products, odour control products, and scented articles.
